# DEMANDE DE BREVET EUROPEEN

(11) **EP 0 889 017 A1**
(43) Date de publication de la demande: **07.01.1999**
(21) Numéro de dépôt: 98202083.6
(22) Date de dépôt: 23.06.1998
(51) Int. Cl.: C07C 19/01, C07C 19/08, C07C 17/278, C07C 17/20, C09K 5/04, C08J 9/14

(54) **Pentachlorobutane, son procédé de fabrication et son utilisation, procédé de préparation du 1,1-difluoro-2-trifluorométhylpropane et utilisation de ce composé**

(30) Priorité: 03.07.1997 BE 9700570
(71) Demandeur: SOLVAY (Société Anonyme), B-1050 Bruxelles (BE)
(72) Inventeur: Bathelemy, Pierre, 1315 Pietrebais (BE); Janssens, Francine, 1800 Vilvoorde (BE); Schoebrechts, Jean-Paul, 5980 Grez-Doiceau (BE); Wilmet, Vincent, 1301 Wavre (BE)
(74) Mandataire: Jacques, Philippe

(57) **Abrégé**

1,1-Dichloro-2-trichlorométhylpropane. Son procédé de synthèse par réaction de télomérisation entre du 1-chloroprop-1-ène et du tétrachlorométhane en présence d'un catalyseur de télomérisation. Son utilisation comme précurseur du 1,1-difluoro-2-trifluorométhylpropane produit par réaction entre du 1,1-dichloro-2-trichlorométhylpropane et du fluorure d'hydrogène, en présence d'un catalyseur d'hydrofluoration.

## Description

La présente invention concerne un nouveau pentachlorobutane (le 1,1-dichloro-2-trichlorométhylpropane) de formule

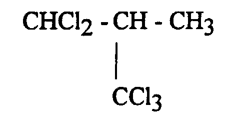

Elle concerne également son procédé de préparation et son utilisation comme précurseur du dérivé pentafluoré correspondant, le 1,1-difluoro-2-trifluorométhylpropane (HFC-365mps).

Le 1,1-difluoro-2-trifluorométhylpropane a été cité dans quelques publications mais aucune n'en divulgue sa préparation.

Le 1,1-difluoro-2-trifluorométhylpropane est un substitut possible des hydrocarbures chlorofluorés entièrement ou partiellement halogénés (CFCs et HCFCs), suspectés d'avoir un effet néfaste sur la couche d'ozone.

La demande de brevet JP 06/41591 décrit une composition pour le nettoyage composée d'un hydrocarbure fluoré contenant au moins 4 atomes de carbone, d'un surfactant et d'un solvant organique. Parmi les hydrocarbures fluorés cités, on trouve le 1,1-difluoro-2-trifluorométhylpropane.

La présente invention a pour objet de fournir un procédé pour la synthèse du 1,1-difluoro-2-trifluorométhylpropane qui met en oeuvre des réactifs couramment ou aisément accessibles et qui présente un rendement élevé, répondant ainsi aux exigences économiques de l'industrie.

En conséquence, la présente invention a pour objet un nouveau pentachlorobutane, le 1,1-dichloro-2-trichlorométhylpropane. Elle concerne également l'utilisation du 1,1-dichloro-2-trichlorométhylpropane comme précurseur du dérivé pentafluoré correspondant, le 1,1-difluoro-2-trifluorométhylpropane par hydrofluoration catalytique. L'invention porte aussi sur un procédé de préparation du 1,1-dichloro-2-trichlorométhylpropane par réaction de télomérisation entre le 1-chloroprop-1-ène et le tétrachlorométhane en présence d'un catalyseur de télomérisation.

Le catalyseur de télomérisation peut être choisi parmi les composés des métaux des groupes 8 à 11 du tableau périodique des éléments (IUPAC 1988) et leurs mélanges. On entend par composés des métaux des groupes 8 à 11, les dérivés organiques et inorganiques de ces métaux. Les composés des métaux des groupes 8 et 11 sont préférés. On retient particulièrement les composés du fer, du nickel et du cuivre; les composés du cuivre (I) ou (II) étant tout particulièrement préférés.

Les composés de cuivre (I) ou (II), utilisés seuls ou en mélange, sont de préférence choisis parmi les halogénures de cuivre, les carboxylates de cuivre, les sels mixtes de cuivre ou les complexes formés avec des ligands neutres.

Parmi les halogénures de cuivre (I) ou (II), on trouve notamment les fluorures, les chlorures, les bromures ou les iodures. Les chlorures et les iodures sont préférés. Le chlorure de cuivre (II) est particulièrement préféré.

Parmi les sels mixtes de cuivre (I) ou (II), on trouve notamment les hydroxyhalogénures. L'hydroxychlorure de cuivre (II) est préféré.

Parmi les carboxylates de cuivre (I) ou (II), on trouve notamment les sels formés au départ d'acides carboxyliques tels que l'acide acétique, l'acide propionique, l'acide butyrique, l'acide cyclohexanebutyrique ou l'acide benzoïque. Les acétates de cuivre (I) ou (II), c'est-à-dire les sels formés aux dépens de l'acide acétique, sont préférés. Le cyclohexanebutyrate de cuivre (II) est tout particulièrement préféré.

Parmi les complexes formés avec des composés du cuivre (I) ou (II), on trouve les complexes formés avec des ligands neutres. A titre d'exemples de ligands neutres, on peut citer les phosphines telles que la triphénylphosphine ou l'acétylacétone. L'acétylacétonate de cuivre (II) est préféré.

Avantageusement, le catalyseur est choisi parmi l'acétate de cuivre (I) ou (II), le cyclohexanebutyrate de cuivre (II), le complexe formé entre le chlorure cuivreux et la triphénylphosphine, l'acétylacétonate de cuivre (II), l'hydroxychlorure de cuivre (II), le chlorure de cuivre (I), l'iodure de cuivre (I) ou le chlorure de cuivre (II). Parmi ces catalyseurs, le chlorure de cuivre (II), l'acétate de cuivre (II), l'hydroxychlorure de cuivre (II), le cyclohexanebutyrate de cuivre (II) et l'acétylacétonate de cuivre (II) sont préférés.

Le procédé de préparation du 1,1-dichloro-2-trichlorométhylpropane selon l'invention peut être effectué en discontinu ou en continu.

Dans un procédé en discontinu, le rapport molaire entre le catalyseur engagé et le 1-chloroprop-1-ène est en général supérieur ou égal à 0,001. Avantageusement, il est supérieur ou égal à 0,005. De préférence, il est supérieur ou égal à 0,01. Le rapport molaire entre le composé de cuivre engagé et le 1-chloroprop-1-ène est habituellement inférieur ou égal à 5. Avantageusement, il est inférieur qu égal à 1. De préférence, il est inférieur ou égal à 0,1.

Dans un procédé en continu, le rapport molaire entre le catalyseur engagé et 1-chloroprop-1-ène évolue approximativement entre les mêmes limites que dans un procédé en discontinu.

Le rapport molaire entre le tétrachlorure de carbone et le 1-chloroprop-1-ène mis en oeuvre peut varier dans de larges mesures. Ce rapport est en général égal ou supérieur à 0,1. Avantageusement, ce rapport est égal ou supérieur à 0,5. D'une manière préférée, il est supérieur ou égal à 1. Généralement, ce rapport est toutefois égal ou inférieur à 20. Avantageusement, ce rapport est égal ou inférieur à 15. D'une manière préférée, ce rapport est égal ou inférieur à 10. D'une manière particulièrement préférée, ce rapport est supérieur ou égal à 2 et inférieur ou égal à 6.

Classiquement, la réaction se déroule à une température supérieure ou égale à la température ambiante. De préférence, la température est égale ou supérieure à 50 °C, avantageusement, la température est égale ou supérieure à 100 °C. En général, la température de réaction ne dépasse pas 250 °C pour éviter toute dégradation des produits formés. Elle est habituellement inférieure ou égale à 200 °C.

La durée de la réaction dans un procédé en discontinu ou le temps de séjour dans un procédé en continu sont fonction de divers paramètres tels que la température de réaction, la concentration en réactifs et en catalyseur dans le mélange réactionnel et leurs rapports molaires. En général, en fonction de ces paramètres, le temps de séjour ou la durée de la réaction peuvent varier de 5 minutes à 40 heures.

La pression est habituellement supérieure ou égale à la pression atmosphérique et égale ou inférieure à 20 bars.

La réaction de télomérisation est généralement réalisée en phase liquide. Elle peut être réalisée en présence d'un solvant et/ou d'un cocatalyseur. Dans un premier mode de mise en oeuvre du procédé selon l'invention, la réaction est réalisée en présence d'un solvant. Avantageusement, le solvant de la réaction est un solvant polaire tel qu'un alcool ou un nitrile. Parmi les alcools utilisables comme solvant pour la réaction, on trouve le méthanol, l'éthanol, l'isopropanol et le tert-butanol. Parmi les nitriles utilisables comme solvant pour la réaction, on trouve les nitriles aliphatiques ou aromatiques. Parmi les nitriles aliphatiques, on trouve l'acétonitrile, le propionitrile, ou l'adiponitrile. Parmi les nitriles aromatiques, on trouve le benzonitrile ou le tolunitrile. De préférence, le solvant est un nitrile. Le propionitrile et l'adiponitrile sont préférés. La quantité de solvant engagé dans la réaction n'est pas critique. Toutefois, une solution trop diluée n'est pas favorable à un rendement et à un taux de conversion élevés. De préférence, le rapport molaire du solvant au 1-chloroprop-1-ène est égal ou supérieur à 0,05. Avantageusement, ce rapport est égal ou supérieur à 0,1. Le rapport molaire du solvant au 1-chloroprop-1-ène est en général égal ou inférieur à 20. Avantageusement, il est égal ou inférieur à 15. D'une manière préférée, ce rapport est égal ou supérieur à 0,2 et égal ou inférieur à 10.

Dans le premier mode de mise en oeuvre, la pression est choisie de façon à maintenir le milieu réactionnel en phase condensée. La pression mise en oeuvre varie en fonction de la température du milieu réactionnel. La pression est habituellement supérieure ou égale à la pression atmosphérique et inférieure ou égale à 15 bars.

Dans un deuxième mode préféré de mise en oeuvre du procédé selon l'invention, la réaction est réalisée en présence d'un cocatalyseur. Le cocatalyseur peut être choisi parmi les amines. Comme amine utilisable, on peut citer les amines aliphatiques ou les amines aromatiques. Parmi les amines aliphatiques, on trouve les amines primaires, les amines secondaires et les amines tertiaires. D'une façon générale, on utilise comme amine les alcanolamines, les alkylamines, comme par exemple l'éthanolamine, la n-butylamine, la tert-butylamine, la n-propylamine, l'isopropylamine, la benzylamine, l'hexaméthylène diamine, la diéthylamine, la triéthylamine ou des amines aromatiques comme la pyridine ou l'aniline. Les alkylamines comme la n-butylamine, la tert-butylamine, la n-propylamine et l'isopropylamine sont préférées. L'isopropylamine et la tert-butylamine sont tout particulièrement préférées.

L'addition d'amine dans le mélange réactionnel n'exclut pas l'utilisation d'un nitrile comme solvant.

Dans ce deuxième mode de mise en oeuvre préféré du procédé selon l'invention, la température de réaction est égale ou inférieure à 200 °C. En particulier, la présence d'amine permet de réaliser la réaction à une température égale ou inférieure à 150 °C tout en conservant un taux de transformation élevé et une excellente sélectivité. Toutefois, la température est supérieure ou égale à 50 °C. Avantageusement, la température est supérieure ou égale à 70 °C. D'une manière particulièrement préférée, la température est généralement supérieure à 80 °C et inférieure à 140 °C.

Le rapport molaire entre le 1-chloroprop-1-ène et le cocatalyseur engagé est en général supérieur ou égal à 0,001. Avantageusement, ce rapport est égal ou supérieur à 0,005. Toutefois, ce rapport est égal ou inférieur à 1. Avantageusement, ce rapport est égal ou inférieur à 0,8. D'une manière préférée, ce rapport est supérieur ou égal à 0.01 et inférieur ou égal à 0,6. D'une manière particulièrement préférée, ce rapport est supérieur ou égal à 0,1 et inférieur ou égal à 0,4. Un excès d'amine peut toutefois conduire à la formation de produits secondaires.

Dans ce deuxième mode de mise en oeuvre du procédé selon l'invention, le tétrachlorure de carbone peut servir à la fois de réactif et de solvant. Le rapport molaire entre le tétrachlorure de carbone et le 1-chloroprop-1-ène est alors supérieur ou égal à 2. Avantageusement, ce rapport est supérieur ou égal à 3. D'une manière préférée, ce rapport est supérieur ou égal à 4. Toutefois, pour éviter une trop grande dilution des réactifs, ce rapport est inférieur ou égal à 10 et de préférence, il est inférieur ou égal à 8. D'une manière préférée, ce rapport est inférieur ou égal à 6.

L'invention se rapporte également à l'utilisation du 1,1-dichloro-2-trichlorométhylpropane pour la préparation du 1,1-difluoro-2-trifluorométhylpropane. Le 1,1-dichloro-2-trichlorométhylpropane peut en effet être converti facilement et avec d'excellents rendements en 1,1-difluoro-2-trifluorométhylpropane par hydrofluoration en présence d'un catalyseur d'hydrofluoration.

L'invention concerne dès lors également un procédé pour la préparation du 1,1-difluoro-2-trifluorométhylpropane selon lequel on fait réagir du 1,1-dichloro-2-trichlorométhylpropane avec du fluorure d'hydrogène, en général en présence d'un catalyseur d'hydrofluoration. Lorsque la réaction est réalisée en phase liquide, la présence d'un solvant organique est envisageable.

Dans le procédé de préparation du 1,1-difluoro-2-trifluorométhylpropane selon l'invention, le catalyseur d'hydrofluoration est avantageusement choisi parmi les dérivés des métaux des groupes 3, 4, 5, 13, 14 et 15 du tableau périodique des éléments (IUPAC 1988) (groupes du tableau périodique des éléments anciennement dénommés IIIa, IVa, IVb, Va, Vb et VIb) et leurs mélanges. On entend par dérivés des métaux, les hydroxydes, les oxydes et les sels organiques et inorganiques de ces métaux ainsi que leurs mélanges. On retient particulièrement les dérivés du titane, du tantale, du molybdène, du bore, de l'étain et de l'antimoine. De préférence, le catalyseur est choisi parmi les dérivés des métaux des groupes 14 (IVa) et 15 (Va) du tableau périodique des éléments et plus particulièrement parmi les dérivés de l'étain et de l'antimoine.

Dans le procédé selon l'invention, les dérivés préférés des métaux sont les sels et ceux-ci sont de préférence choisis parmi les halogénures, et plus particulièrement parmi les chlorures, les fluorures et les chlorofluorures. Des catalyseurs d'hydrofluoration particulièrement préférés selon la présente invention sont les chlorures, les fluorures et les chlorofluorures d'étain et d'antimoine.

Dans le cas où le catalyseur est sélectionné parmi les fluorures et les chlorofluorures métalliques, ceux-ci peuvent être obtenus au départ d'un chlorure que l'on soumet à une fluoration au moins partielle. Cette fluoration peut par exemple être réalisée au moyen de fluorure d'hydrogène, préalablement à la mise en contact du catalyseur avec le 1,1-dichloro-2-trichlorométhylpropane . En variante, elle peut être réalisée in situ, au cours de la réaction du 1,1-dichloro-2-trichlorométhylpropane avec le fluorure d'hydrogène.

La quantité de catalyseur mise en oeuvre peut varier dans de larges limites. Elle est généralement d'au moins 0,001 mole de catalyseur par mole de 1,1-dichloro-2-trichlorométhylpropane . De préférence, elle est d'au moins 0,01 mole de catalyseur par mole de 1,1-dichloro-2-trichlorométhylpropane . En principe, il n'y a pas de limite supérieure à la quantité de catalyseur mise en oeuvre. Par exemple, dans un procédé réalisé en continu, en phase liquide, le rapport molaire entre le catalyseur et le 1,1-dichloro-2-trichlorométhylpropane peut atteindre 1000. En pratique toutefois, on utilise généralement au maximum environ 5 moles de catalyseur par mole de 1,1-dichloro-2-trichlorométhylpropane. De préférence, on ne dépasse pas environ 1 mole. De manière particulièrement préférée, on ne dépasse pas environ 0,8 mole de catalyseur par mole de 1,1-dichloro-2-trichlorométhylpropane.

Le rapport molaire entre le fluorure d'hydrogène et le 1,1-dichloro-2-trichlorométhylpropane mis en oeuvre est généralement d'au moins 5. De préférence on travaille avec un rapport molaire d'au moins 8. Le rapport molaire entre le fluorure d'hydrogène et le 1,1-dichloro-2-trichlorométhylpropane mis en oeuvre ne dépasse en général pas 200. De préférence, il ne dépasse pas 100.

La température à laquelle s'effectue l'hydrofluoration est généralement d'au moins 50 °C. De préférence, elle est d'au moins 80 °C. La température ne dépasse en général pas 150 °C. De préférence, elle ne dépasse pas 130 °C.

Le procédé de préparation du 1,1-difluoro-2-trifluorométhylpropane est de préférence réalisé en phase liquide. Dans ce cas, la pression est choisie de manière à maintenir le milieu réactionnel sous forme liquide. La pression mise en oeuvre varie en fonction de la température du milieu réactionnel. Elle est généralement de 2 à 40 bars. De manière préférée, on travaille à une température et à une pression auxquelles, en outre, le 1,1-difluoro-2-trifluorométhylpropane produit est au moins partiellement sous forme gazeuse, ce qui permet de le séparer aisément du milieu réactionnel.

Le procédé de préparation du 1,1-difluoro-2-trifluorométhylpropane selon l'invention peut être mis en oeuvre de manière continue ou discontinue. Il est entendu que la quantité de catalyseur mise en oeuvre est exprimée, dans un procédé discontinu, par rapport à la quantité initiale de 1,1-dichloro-2-trichlorométhylpropane mise en oeuvre et, dans un procédé continu, par rapport à la quantité stationnaire de 1,1-dichloro-2-trichlorométhylpropane présent dans la phase liquide.

Le temps de séjour des réactifs dans le réacteur doit être suffisant pour que la réaction du 1,1-dichloro-2-trichlorométhylpropane avec le fluorure d'hydrogène se réalise avec un rendement acceptable. Il peut être déterminé aisément en fonction des conditions opératoires retenues.

Le procédé selon l'invention peut être réalisé dans tout réacteur construit en un matériau résistant à la température, à la pression et aux réactifs utilisés, notamment au fluorure d'hydrogène. Il est avantageux de séparer du milieu réactionnel le 1,1-difluoro-2-trifluorométhylpropane et le chlorure d'hydrogène au fur et à mesure qu'ils se forment et de maintenir ou de renvoyer dans le réacteur les réactifs non transformés, ainsi que les chlorofluorobutanes éventuellement formés par fluoration incomplète du 1,1-dichloro-2-trichlorométhylpropane. A cet effet, le procédé selon l'invention est avantageusement réalisé dans un réacteur équipé d'un dispositif de soutirage d'un courant gazeux, ce dispositif consistant par exemple en une colonne de distillation et un condenseur de reflux surmontant le réacteur. Ce dispositif permet, par un réglage adéquat, de soutirer en phase gazeuse le 1,1-dichloro-2-trichlorométhylpropane et la majeure partie du fluorure d'hydrogène non transformés, ainsi que, le cas échéant, la majeure partie des produits de fluoration partielle du 1,1-dichloro-2-trichlorométhylpropane.

Les exemples ci-après illustrent l'invention de manière non limitative.

Dans les exemples ci-dessous, le taux de conversion est le rapport entre la concentration molaire initiale en 1-chloroprop-1-ène diminuée de sa concentration molaire finale divisée par la concentration molaire initiale, multiplié par 100; la sélectivité en 1,1-dichloro-2-trichlorométhylpropane est le rapport entre la concentration molaire finale en 1,1-dichloro-2-trichlorométhylpropane divisée par la concentration molaire initiale en 1-chloroprop-1-ène diminuée de sa concentration molaire finale, multiplié par 100; le rendement en 1,1-dichloro-2-trichlorométhylpropane est le rapport entre la concentration molaire finale en 1,1-dichloro-2-trichlorométhylpropane divisé par la concentration molaire initiale en 1-chloroprop-1-ène multiplié par 100.

### Exemple 1: (conforme à l'invention)

Dans cet exemple, on a préparé du 1,1-dichloro-2-trichlorométhylpropane par réaction entre du 1-chloroprop-1-ène et du tétrachlorométhane en présence d'un catalyseur constitué d'un mélange contenant 20 % de chlorure de cuivre (I) et 80 % d'hydroxychlorure de cuivre (II).

Dans un autoclave de 300 ml dont les parois internes sont recouvertes d'une résine fluorocarbonée, on a introduit 5,4 ml de 1-chloroprop-1-ène, 25,8 ml de tétrachlorométhane, 0,46 g de catalyseur et 18,8 ml de propionitrile soit un rapport molaire de 1/4/0,07/4.

L'appareil a été fermé hermétiquement, placé dans un four vertical et la température a été augmentée progressivement et maintenue à 160 °C pendant 20 heures. L'agitation est assurée par un barreau magnétique placé dans le fond de l'autoclave. En fin de réaction, on a laissé refroidir l'autoclave, un échantillon de liquide a été prélevé à la seringue et dosé par une méthode chromatographique pour déterminer le taux de conversion du 1-chloroprop-1-ène et la sélectivité en 1,1-dichloro-2-trichlorométhylpropane. On obtient ainsi du 1,1-dichloro-2-trichlorométhylpropane avec un rendement de 60 %, un taux de conversion de 92 % et une sélectivité de 65 %.

### Exemple 2 : (conforme à l'invention)

Dans cet exemple, on a préparé du 1,1-difluoro-2-trifluorométhylpropane à partir de 1,1-dichloro-2-trichlorométhylpropane et de fluorure d'hydrogène en présence de pentachlorure d'antimoine à titre de catalyseur.

Dans un autoclave de 0,5 1 en acier inoxydable HASTELLOY B2, équipé d'un agitateur mécanique à pales, d'une sonde de température et d'un tube plongeant permettant d'effectuer des prélèvements en phase liquide en cours d'essai, on a introduit 26,5 g de 1,1-dichloro-2-trichlorométhylpropane (115 mmoles), 8 g de pentachlorure d'antimoine (26,4 mmoles) et 207 g de fluorure d'hydrogène (10,35 moles). L'autoclave a ensuite été plongé dans un bain thermostatisé, maintenu à une température de 120 °C sous agitation continue durant 48 heures. La pression dans le réacteur a été régulée à 25 bar. Un prélèvement réalisé après 12 heures a montré que la conversion du 1,1-dichloro-2-trichlorométhylpropane était totale dont 8 % en 1,1-difluoro-2-trifluorométhylpropane. Après 48 heures de réaction, le rendement en 1,1-difluoro-2-trifluorométhylpropane était de 50 %.

### Exemple 3: (conforme à l'invention)

Le même mode opératoire que dans l'exemple 2 a été appliqué mais en remplaçant le pentachlorure d'antimoine par 15 g (57,6 mmoles) de tétrachlorure d'étain. Dans ce cas, le rendement en 1,1-difluoro-2-trifluorométhylpropane est de 50 % après 20 heures et de 95 % après 50 heures de réaction.

### Exemple 4 : (conforme à l'invention)

On a préparé un prémélange pour la fabrication d'une mousse de polyisocyanurate en mélangeant (quantités pondérales) :
- 100 parties de polyéther polyol
- 1 partie d'eau
- 3 parties de surfactant siliconé
- 3 parties de diméthylcyclohexylamine
- 7 parties de KACEKAT® KCA (catalyseur - SOLVAY)
- 60 parties de HFC-365mps.

On a caractérisé le profil de réactivité de ce prémélange en mesurant les paramètres suivants :
- temps de crème : temps à l'issue duquel on observe un changement de coloration du mélange et qui correspond au début de la montée en mousse
- temps de fil : temps à partir duquel on commence à observer la formation de fils de polymère lorsqu'une baguette de verre est mise en contact avec la mousse
- temps de montée : temps mis par la mousse pour atteindre sa hauteur maximale
- temps hors colle : temps à partir duquel la mousse n'est plus collante.

On obtient ainsi une mousse de PIR ayant un temps de crème de 26 secondes, un temps de fil de 69 secondes, un temps de montée de 96 secondes et un temps hors colle de 80 secondes avec une densité de 38,2 kg/m³.

## Revendications

1. 1,1-dichloro-2-trichlorométhylpropane de formule

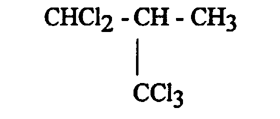

2. Procédé de préparation du 1,1-dichloro-2-trichlorométhylpropane par réaction entre du 1-chloroprop-1-ène et du tétrachlorométhane en présence d'un catalyseur de télomérisation.

3. Procédé selon la revendication 2 dans lequel le catalyseur de télomérisation est choisi parmi les composés des métaux des groupes 8 à 11 du tableau périodique des éléments (IUPAC 1988) et leurs mélanges et, en particulier, parmi l'acétate de cuivre (II), l'hydroxychlorure de cuivre (II), le cyclohexanebutyrate de cuivre (II), l'acétylacétonate de cuivre (II) et le chlorure de cuivre (II).

4. Procédé selon la revendication 2 ou 3 dans lequel la réaction est réalisée en présence d'un solvant et/ou d'un cocatalyseur.

5. Procédé selon la revendication 4 dans lequel le solvant est choisi parmi l'acétonitrile, le propionitrile et l'adiponitrile et le cocatalyseur est choisi parmi l'isopropylamine et la tert-butylamine.

6. Procédé pour la préparation de 1,1-difluoro-2-trifluorométhylpropane, selon lequel on fait réagir du 1,1-dichloro-2-trichlorométhylpropane avec du fluorure d'hydrogène en présence d'un catalyseur d'hydrofluoration.

7. Procédé selon la revendication 6, dans lequel le catalyseur d'hydrofluoration est choisi parmi les dérivés des métaux des groupes 3, 4, 5, 13, 14 et 15 du tableau périodique des éléments (IUPAC 1988) et leurs mélanges, en particulier parmi les chlorures, les fluorures et les chlorofluorures d'étain et d'antimoine.

8. Procédé selon la revendication 6 ou 7, dans lequel le catalyseur est mis en oeuvre en une quantité de 0,01 à 1 mole de catalyseur par mole de 1,1-dichloro-2-trichlorométhylpropane et le fluorure d'hydrogène est mis en oeuvre en une quantité de 5 à 100 moles de fluorure d'hydrogène par mole de 1,1-dichloro-2-trichlorométhylpropane et dans lequel on réalise la réaction à une température d'environ 50 à 150 °C et à une pression de 2 à 40 bar.

9. Utilisation du 1,1-dichloro-2-trichlorométhylpropane comme précurseur dans la synthèse du 1,1-difluoro-2-trifluorométhylpropane.

10. Utilisation du 1,1-difluoro-2-trifluorométhylpropane comme aérosol, comme réfrigérant et comme agent gonflant pour mousses cellulaires polymériques.
